# EUROPEAN PATENT APPLICATION

(11) **EP 1 323 433 A1**
(43) Date of publication of application: **02.07.2003**
(21) Application number: 01938690.3
(22) Date of filing: 15.06.2001
(51) Int. Cl.: A61K 49/06, A61K 31/198, A61K 31/663, A61K 33/24, A61K 47/26, A61K 47/30, A61K 47/42, A61P 13/04

(54) **COMPOSITIONS FOR PREVENTING URINARY CALCULUS**

(30) Priority: 04.08.2000 JP 2000237329
(71) Applicant: Nihon Medi-Physics Co., Ltd., Nishinomiya-shi, Hyogo 662-0918 (JP)
(72) Inventor: YOSHIDA, Yasunori, Sodegaura-shi, Chiba 299-0266 (JP)
(74) Representative: Pohlmann, Eckart, Dipl.-Phys.
(86) International application number: JP0105125
(87) International publication number: WO02011770

(57) **Abstract**

Provided is a composition for preventing urinary calculi caused by metal ions liberated from a compound containing a lanthanoid metal or another heavy metal in image diagnosis or therapy using that compound. The composition is a urinary calculus preventing composition containing a multidentate ligand capable of forming a complex with lanthanoid metal ions or other heavy metal ions, or an alkaline earth metal complex of that ligand. The multidentate ligand can be a bifunctional ligand having a polyaminopolycarboxylic acid, polyaminopolyphosphonic acid or the like as its site for forming a complex with a metal and chemically bonded to a physiologically acceptable oligosaccharide or the like, or can also be a polyaminopolycarboxylic or polyaminopolyphosphonic acid per se. Preferably, the polyaminopolycarboxylic acid is EDTA, DTPA, DOTA or TETA, or a derivative thereof.

## Description

### Technical Field

The present invention relates to a composition for preventing urinary calculi, more particularly, a composition for preventing urinary calculi that are caused by metal ions liberated from a lanthanoid metal-containing compound or another heavy metal-containing compound in image diagnosis or therapy using said compound.

### Background Art

In recent years, contrast media containing a lanthanoid metal have been generally used in the field of image diagnosis, especially magnetic resonance imaging (MRI). The lanthanoid metal-containing MRI contrast media used as medical products are presently limited to those containing gadolinium such as a complex of diethylenetriaminepentaacetic acid with trivalent gadolinium ion (Gd-DTPA) and a complex of diethylenetriaminepentaacetic acid bismethylamide with trivalent gadolinium ion (Gd-DTPA-BMA). However, as can be seen in JP10-511934A (corresponding to WO96/16928) for example, there are attempts for applying other lanthanoid metals such as dysprosium. Also, said JP10-511934A (corresponding to WO96/16928) directs attention to X-ray absorption characteristics of bismuth- and other heavy metal-containing compounds and lanthanoid metal-containing compounds, and indicates that these compounds may be used as X-ray contrast media instead of iodine-containing X-ray contrast media. Moreover, as can be seen in JP6-55681B (corresponding to EP164843A2) for example, use of heavy metals to radionuclide-based therapeutic agents for internal radiation is also proposed.

The lanthanoid metals and other heavy metals in these agents are held in carriers by way of such bonds as coordinate bonds that are chemically weaker than covalent bonds. Therefore, they are likely to be liberated from carriers in vivo due to competition with other ions. It is indicated that, in general, if lanthanoid metal ions are liberated in vivo, especially in blood, they act as heavy metal ions (Cacheris WP, Quay SC and Rocklage RC: Magnetic Resonance Imaging, 8, 467-481, 1990). It can be predicted that heavy metal ions other than lanthanoid metal ions also act similarly. However, behavior of lanthanoid metal-containing pharmaceuticals and of liberated lanthanoid metal ions after their migration from blood into urine, and their effects on living bodies have not been particularly discussed yet.

The inventor has recently carried out toxicity studies by repeated administration to rats of a gadolinium ion-chelated compound (hereinafter abbreviated as R-CHI3-DTPA-Gd) which was prepared by allowing diethylenetriaminepentaacetic anhydride to react with amino groups of chitosan trimer (hereinafter abbreviated as R-CHI3) having a reduction-treated reducing end to yield a compound (hereinafter abbreviated as R-CHI3-DTPA) bonded with diethylenetriaminepentaacetic acid, followed by chelation. In the study, the inventor has found urinary calculi very frequently, and investigated the cause.

As a result, it has been found that the calculi are composed of ordinary calculus components such as phosphoric acid, calcium and magnesium besides a slight amount of gadolinium. This has led to a conclusion that a slight amount of lanthanoid metal ions liberated for a certain reason in blood or the renal/urinary system are combined in the urine with a urine component, namely phosphoric acid, to form a sparingly soluble phosphate which, in turn, acts as a nucleus and grows into a calculus mainly composed of calcium phosphate, magnesium phosphate and the like. This conclusion can be applied not only to lanthanoid metals but also to other heavy metals capable of forming phosphates sparingly soluble or insoluble in water.

The thus-formed calculus is not different from the naturally produced urinary calculus at all, and thus it is considered that the calculus will, in some cases, be naturally discharged, or in some other cases, remain at a specific region to cause a clinical symptom mentioned below. Namely, in the case where the formed calculus is an upstream urinary calculus such as a renal calculus existing in the renal parenchyma, calyx or renal pelvis or a urinary calculus existing in the ureter, a symptom such as hematuria or dorsolumbar pain may happen, and in the case where the formed calculus is a downstream urinary calculus such as a vesical calculus existing in the bladder or a urethral calculus existing in the urethra, a symptom such as hematuria, pain or cystitis may happen.

The present invention provides a composition for preventing urinary calculi caused by a metal ion that is liberated from a compound containing a lanthanoid metal or another heavy metal in image diagnosis or therapy using said compound.

### Disclosure of the Invention

The inventor has intensively researched measures for prevention of calculi based on the above-mentioned mechanism of calculus generation, and as a result, has found that frequency of the calculus generation declines when compounds that contain lanthanoid metals or other heavy metals for administration in image diagnosis, therapy or the like coexist with a multidentate ligand capable of forming a complex with ions of said metals. Based on this finding, the present invention has been completed.

That is, the present invention provides a composition for preventing urinary calculi, which comprises a multidentate ligand capable of forming a complex with lanthanoid metal ions or other heavy metal ions, or an alkaline earth metal complex of said ligand. Thus, if the composition of the present invention is administered in vivo so that it exists concurrently with a substance that will cause urinary calculi in the renal/urinary system, lanthanoid metal ions or other heavy metal ions liberated from the substance are captured by the ligand in the composition of this invention, whereby inhibition of formation of calculus nuclei is effected, and thus generation of calculi is prevented.

The composition of the present invention contains, as a major ingredient, a multidentate ligand capable of forming a complex with lanthanoid metal ions or other heavy metal ions. The multidentate ligand referred to herein is a compound capable of forming a stable complex with lanthanoid metal ions or other heavy metal ions, and may be either a multidentate ligand per se or a compound containing the multidentate ligand in its structure.

The multidentate ligand is preferably a chained or cyclic polyaminopolycarboxylic acid, or a chained or cyclic polyaminopolyphosphonic acid. Examples of the former include ethylenediaminediacetic acid, nitrilotriacetic acid, ethylenediaminetetraacetic acid (hereinafter abbreviated as EDTA), diaminocyclohexanetetraacetic acid, diethylenetriaminepentaacetic acid (hereinafter abbreviated as DTPA), triethylenetetraminehexaacetic acid, 1,4,7,10-tetraazacyclododecane-1,4,7,10-tetraacetic acid (hereinafter abbreviated as DOTA), 1,4,8,11-tetraazacyclotetradecane-1,4,8,11-tetraacetic acid (hereinafter abbreviated as TETA), and their derivatives. Examples of the latter include ethylenediaminetetrakismethylenephosphonic acid (hereinafter abbreviated as EDTMP) and its derivatives.

Examples of derivatives of polyaminopolycarboxylic acids include compounds obtained by esterifying or halogenating one or plural carboxyl groups of a polyaminopolycarboxylic acid, or adding a protective group to the carboxyl group(s), or substituting the carboxyl group(s) with a hydrocarbon group having a substituent group other than carboxylic acid, compounds obtained by introducing a hydrocarbon group having a substituent group other than carboxylic acid or introducing a substituent group containing no hydrocarbon into the hydrocarbon portion of a polyaminopolycarboxylic acid, and compounds obtained by introducing, for example, an ether group to the carbon skeleton portion of a polyaminopolycarboxylic acid. Concrete examples thereof include hydroxyethylethylenediaminetriacetic acid, diaminopropanoltetraacetic acid, N,N-bis(2-hydroxybenzyl)ethylenediaminediacetic acid, glycoletherdiaminetetraacetic acid, etc.

The compound containing a multidentate ligand in its structure can be a compound having a bifunctional ligand chemically bonded to a physiologically acceptable monosaccharide, oligosaccharide, polysaccharide, amino acid, oligopeptide, polypeptide, nucleotide, oligonucleotide, polynucleotide, protein, protein fragment or chemical derivative thereof or synthetic polymer, etc. The above-mentioned physiologically acceptable compounds ranging from monosaccharide through synthetic polymer are hereinafter called "physiologically acceptable substances".

The bifunctional ligand is a compound that has, in its molecule, both a site for bonding to a physiologically acceptable substance and a site for forming a complex with a metal. Therefore, by way of functional groups that are present in the molecule of a physiologically acceptable substance and available for bonding to bifunctional ligands, bifunctional ligands as many as the functional groups can be chemically bonded to the physiologically acceptable substance.

The site for forming a complex with a metal is not especially limited as long as it is a multidentate ligand capable of forming a stable complex with each metal, and usually, it can be selected from the above-mentioned cyclic or chained polyaminopolycarboxylic acids and cyclic or chained polyaminopolyphosphonic acids. For example, EDTA, DTPA, DOTA, TETA or their derivatives, or EDTMP or its derivatives can be used. In addition to them, 6-hydrazinonicotinic acid or a series of multidentate ligands containing sulfur and nitrogen as coordinating atoms such as diaminodithiols, monoaminomonoamidodithiols, diamidodithiols and triamidothiols can also be used as the site for forming a complex. They are exemplified by the following chelate groups. As the diaminodithiols, mention may be made of N,N'-bis(2-mercaptoethyl)ethylenediamine and 2,2,9,9-tetramethyl-4,7-diaza-1,10-decanethiol. As the monoamidomonoaminodithiols, mention may be made of N-2-mercaptoethyl-2-mercaptoethylaminoacetamide and N-(2-mercaptoethyl)aminoethyl-2-mercaptoacetamide. As the diamidodithiols, mention may be made of 1,2-ethylenebis(2-mercaptoacetamide). As the triamidothiols, mention may be made of mercaptoacetylglycylglycylglycine.

The site for bonding to a physiologically acceptable substance is constituted by a reactive bonding group of the bifunctional ligand, which is exemplified by ordinary amino group, carboxyl group and thiol group as well as active halogens, alkoxy esters, N-hydroxysuccinimide esters, imide esters, maleimides, thiophthalimides, isothiocyanates and acid anhydrides.

Examples of the bifunctional ligand include compounds with a polyaminopolycarboxylic acid or polyaminopolyphosphonic acid as a site for forming a complex with a metal, such as 1-(p-isothiocyanatobenzyl)-DTPA (Martin, WB, et al., Inorg. chem., 25, pages 2772-2781, 1986), DTPA anhydride, 2-(p-isothiocyanatobenzyl)-1,4,7,10-tetraazacyclododecane-1,4,7,10-tetraacetic acid (US Patent No. 4678667), succinimidyl-6-hydrazinonicotinate (Abrams, M.J., et al., J. Nucl. Med., 31, pages 2022-2028, 1990), DTPA-mono(2-aminoethylamide), DTPA-mono(3-aminopropylamide), DTPA-mono(6-aminohexylamide) (Japanese Patent No. 2815615 (corresponding to EP345723B1), 1-(4-aminobenzyl)-EDTA, 1-(4-isothiocyanobenzyl)-EDTA, 1-[4-(3-maleimidopropyl)amidobenzyl]-EDTA, and 1-[4-(5-maleimidopentyl)amidobenzyl]-EDTA.

The bonding between a physiologically acceptable substance and a bifunctional ligand can be achieved by known methods. For example, the reaction between a physiologically acceptable substance and a bifunctional ligand having an acid anhydride (Hnatowich, DJ, et al., Int. J. Appl. Rad. Isot., 33, pages 327-332, 1982), an isothiocyanate (Esteban, JM, et al., J. Nucl. Med., 28, pages 861-870, 1987), an alkoxy ester (Washburn, LC, et al., Nucl. Med. Biol., 18, pages 313-321, 1991) or an active halogen (Fourie, PJ, et al., Eur. J. Nucl. Med., 4, pages 445-448, 1979) as a reactive bonding group, can be carried out as described in known documents cited above. An alkaline earth metal can be complexed to said bifunctional ligand according to ordinary methods.

Furthermore, said bifunctional ligand can be connected with a physiologically acceptable substance by way of a crosslinking agent. The crosslinking agent can be a divalent reagent used for enzyme immunoassay, etc. Examples of the crosslinking agent include N-succinimidyl-3-(2-pyridyldithio)propionate (SPDP), ethylene glycol-O,O'-bis(succinimidyl succinate) (EGS), N-(4-maleimidobutyryloxy)succinimide (GMBS), N-(4-maleimidobutyryloxy)sulfosuccinimide sodium salt (Sulfo-GMBS), N-(6-maleimidocaproyloxy)sulfosuccinimide sodium salt (Sulfo-EMCS), N-(8-maleimidocarpyloxy)sulfosuccinimide sodium salt (Sulfo-HMCS), N-(11-maleimidoundecanoyloxy)sulfosuccinimide sodium salt (Sulfo-KMUS), 3,3'-dithiobis(sulfosuccinimidyl propionate) (DTSSP), glutaraldehyde, etc.

The reaction between said crosslinking agent and said physiologically acceptable substance and the reaction between said crosslinking agent and said bifunctional ligand can be carried out according to known methods. For example, the reaction for bonding DTPA-mono(2-aminoethylamide) or DTPA-mono(6-aminohexylamide) to the amino group of IgG or Fab fragment by way of EGS or DTSSP can be carried out according to the method of Japanese Patent No. 2815615 (corresponding to EP345723B1).

Moreover, said bifunctional ligand can be connected with the physiologically acceptable substance by way of a reactive polymer such as polylysine, polyacrolein, dialdehyde starch or amino oligosaccharide. In this case, the reactive polymer and the bifunctional ligand may be connected with each other directly or by way of a crosslinking agent. This applies also to the bonding between the physiologically acceptable substance and the reactive polymer. Since such a reactive polymer has many reactive groups such as amino groups capable of bonding to said bifunctional ligand or crosslinking agent, the quantity of the ligand per the physiologically acceptable substance can be appropriately adjusted.

The reaction between said reactive polymer and said physiologically acceptable substance and the reaction between said reactive polymer and said bifunctional ligand or crosslinking agent can be carried out by known methods. For example, the reaction can be effected using polylysine in accordance with Japanese Patent No. 2548711 (corresponding to EP233619B1); using polyacrolein in accordance with Japanese Patent No. 1729192 (corresponding to EP111311A2); using dialdehyde starch in accordance with Japanese Patent No. 1721409 (corresponding to EP111311A2); and using amino oligosaccharide in accordance with JP7-206895A (corresponding to EP649857A1).

Among said compounds containing a multidentate ligand in the structure thereof, preferred compounds include a compound in which at least one bifunctional ligand is chemically bonded to an amino group of an amino oligosaccharide having a molecular weight of 500 to 2000 and having a reduction-treated reducing end, and a compound in which at least one bifunctional ligand is chemically bonded to an aldehyde group of a dialdehyde-oligosaccharide which has at least one constitutional monosaccharide that is oxidation-cleaved and has a molecular weight of 500 to 2000 with a reduction-treated reducing end, as described in JP8-208525A (corresponding to EP707857A1).

Preferred amino oligosaccharides include chitosan oligosaccharide and galactosamine oligosaccharide, of trimer, tetramer, pentamer, hexamer, heptamer, octamer, nonamer and decamer. Especially preferred are chitosan oligosaccharides and galactosamine oligosaccharides, of trimer, tetramer, pentamer and hexamer.

Preferred dialdehyde-oligosaccharides include dialdehydesaccharides such as maltotriose, maltotetraose, maltopentaose, maltohexaose, maltoheptaose, isomaltotriose, isomaltotetraose, isomaltopentaose, isomaltohexaose, isomaltoheptaose, cellotriose, cellotetraose, cellopentaose, cellohexaose, laminaritriose, laminaritetraose, laminaripentaose, laminarihexaose, laminariheptaose, elrose, panose, raffinose, etc.

The multidentate ligands may be used in a free or salt form as a main ingredient of the urinary calculus preventing composition. Also, it may be used in a form of an alkaline earth metal complex for the purpose of avoiding any effect that may be caused by formation of a complex of the multidentate ligand with internal metal ions. In case a multidentate ligand is used as an alkaline earth metal complex for this purpose, magnesium or calcium is preferred as the alkaline earth metal.

Furthermore, the multidentate ligands and their alkaline earth metal complexes may be used alone, or as a mixture of two or more appropriately combined, for example, by blending some kinds of multidentate ligands together, or blending some kinds of multidentate ligands with their alkaline earth metal complexes, considering the excretion rate into the renal/urinary system and toxicity.

It is only required that the multidentate ligand or its alkaline earth metal complex exists concurrently with a causal substance for a calculus in the renal/urinary system, and thus administration route into living bodies and administration timing can be decided, considering in vivo kinetics and stability thereof. As examples, mention may be made of not only general routes such as intravascular administration, oral administration and intraperitoneal administration, but also direct injection utilizing a technique of retrograde pyelography. However, in view of simplicity and efficacy, it is preferred to administer intravenously before, after or simultaneously with administration of a lanthanoid- or heavy metal-containing compound. Irrespective of administration route, when the multidentate ligand or its alkaline earth metal complex is administrated simultaneously with the lanthanoid- or heavy metal-containing compound, they can also be mixed with each other immediately before they are used.

As for the dose, even if it is small, the effect of inhibiting formation of calculus nuclei can be expected, as can be seen also in the working examples described later. The upper limit of the dose should be decided depending upon each multidentate ligand based on a toxicity test and a test for proving potency. For example, in the case of DTPA, as can be seen in the case where it was used for promoting discharge of radioactive substances that have been ingested into the body (Shun Fukuda: Radiation Chemistry, 32, 115-118, 1989), about 1000 mg/70 kg body weight is a realistic upper limit of the dose. Since the dose of an MRI contrast medium frequently used at present is 0.1 mmol/kg body weight as gadolinium, the afore-said dose of DTPA (1000 mg/70 kg = 0.036 mmol/kg) corresponds to about 36% of the dose of gadolinium. From these matters, it is preferred that the dose of a multidentate ligand, its alkaline earth metal complex, or their mixture is in a range of 0.1 to 30 mol% as the net multidentate ligand based on the total amount of the lanthanoids or heavy metals administered per time for the purpose of diagnosis or therapy. Furthermore, for example, when an MRI contrast medium is mixed together immediately before use, gentle trans-chelation of components from the MRI contrast medium into the multidentate ligand may be anticipated as the case may be. In such a case, it is preferred that the dose of the multidentate ligand compound, its alkaline earth metal complex or their mixture is kept in a range of 0.1 to 10 mol% as the net multidentate ligand based on the total amount of the lanthanoids or heavy metals administered per time for the purpose of diagnosis or therapy.

The urinary calculus preventing composition may contain pharmaceutically acceptable additives such as a buffer as an ingredient for pH regulation and sodium chloride for isotonization, and can be processed into a formulation suitable for an administration route, such as a liquid or freeze-dried product. Furthermore, a composition may be a preparation obtained by mixing a multidentate ligand or its alkaline earth metal complex with a pharmaceutical composed of a lanthanoid metal-containing compound or a heavy metal-containing compound.

### Reference Example 1

### Test of repeated administration of R-CHI3-DTPA-Gd to rats

A chitosan trimer (R-CHI3) having a reduction-treated reducing end was bonded at an amino group thereof to diethylenetriaminepentaacetic acid to obtain a compound (R-CHI3-DTPA), and it was further chelated with gadolinium to yield a compound (R-CHI3-DTPA-Gd) according to the method disclosed in Example 1 of JP8-208525A (corresponding to EP707857A1). According to the Guideline for Drug Toxicity Testing Methods and GLP standard, the obtained R-CHI3-DTPA-Gd was subjected to toxicity studies by repeated administration intravenously at doses of 0.013, 0.033, 0.1 and 0.33 mmol/kg (0.04, 0.1, 0.3, 1 mmol/kg as DTPA-Gd) to male rats for 4 weeks. To the control group, physiological saline was administered.

Frequency of occurrence of calculi after completion of administration is shown in Table 1.

As shown in Table 1, the frequency of occurrence of calculi became higher with increase of dose, and at the largest dose, calculi were observed in 10 rats out of 18 rats. Also, in the control group, one rat showed calculi, but this should be interpreted as a spontaneous occurrence. Obviously, it should be considered that the increase in the frequency of occurrence of calculi with the increase of dose is attributed to the administration of R-CH13-DTPA-Gd.

**Table 1**

| Calculus occurring frequency | | | | | |
|---|---|---|---|---|---|
| Dose* | 0(control) | 0.013 | 0.033 | 0.1 | 0.33 |
| Frequency** | 1/18 | 1/12 | 4/12 | 6/12 | 10/18 |

| | | | | | |
|---|---|---|---|---|---|
| *Dose: mmol/kg | | | | | |
| **Frequency: the number of individuals showing a calculus/the number of all individuals of each group | | | | | |

### Reference Example 2

### Component analysis of calculi

The calculi obtained from the individuals of the control group and the largest dose group of Reference Example 1, were analyzed to identify their component elements and ion species. For analyzing the component elements, each calculus was dissolved into nitric acid and semi-quantitatively determined by means of ICP-MS (inductively coupled plasma-mass analysis). The results are shown in Table 2. For ion species, each calculus was dissolved into an acid, and capillary electrophoresis and ion chromatography were used to quantitatively determine the ion species shown in Table 3.

The amount of analyzed specimens was slight, and determination of addition/recovery rates was impossible. Therefore, no qualitative discussion is possible. However, as can be seen from Tables 2 and 3, the calculi formed in the individuals administered with R-CHI3-DTPA-Gd obviously contained gadolinium in addition to ordinary calculus components such as phosphoric acid, magnesium, calcium, ammonium, etc. On the other hand, no gadolinium was detected in the calculi formed in the individual of the control group. From these results of analysis, it has been clarified that gadolinium originating from R-CHI3-DTPA-Gd would participate in the formation of calculi.

**Table 2**

| Analysis of component elements of calculi | | |
|---|---|---|
| Relative amount (µg/g calculus) | Calculi from the largest dose group | Calculi from the control group |
| 1000∼ | Mg | Na |
| 100∼1000 | Gd, Na, Ca | - |
| 10∼100 | Br | - |
| ∼10 | Zn, Mn etc | Mg, Ca etc |

**Table 3**

| Analysis of ion species of calculi | | |
|---|---|---|
| Ion species | Relative amount of ions (mmol/g calculus) | |
| | Calculus #1 from the largest dose group | Calculus #2 from the largest dose group |
| Phosphoric acid | 1.6 | 0.92 |
| Citric acid | ND | ND |
| Oxalic acid | ND | ND |
| Uric acid | ND | ND |
| Magnesium | 1.6 | 1.0 |
| Calcium | ND | 0.07 |
| Sodium | 0.2 | 0.34 |
| Ammonium | 1.0 | 0.09 |
| Potassium | 0.3 | 0.04 |

### Example 1

### Calculus prevention effect by chelating agents

To R-CHI3-DTPA-Gd, was added diethylenetriaminepentaacetic acid (DTPA), its calcium complex, or calcium complex of ethylenediaminetetraacetic acid (EDTA). Using them as specimens, toxicity studies by repeated intravenous administration were conducted on male rats for 2 weeks. A group, which is called DTPA-10 group, was administered with a specimen of R-CHI3-DTPA-Gd mixed with DTPA in an amount equivalent to 10 mmol per 500 mmol of the DTPA-Gd portion of the R-CHI3-DTPA-Gd; a group, which is called DTPA-Ca-10 group, was administered with a specimen mixed with calcium complex of DTPA in an amount equivalent to 10 mmol; a group, which is called EDTA-Ca-1 group, was administered with a specimen mixed with calcium complex of EDTA in an amount equivalent 1 mmol; and a group, which is called EDTA-Ca-10 group, was administered with a specimen mixed with calcium complex of EDTA in an amount equivalent to 10 mmol. DTPA and others were mixed with R-CHI3-DTPA-Gd immediately before administration. As control groups, were set up a group that was administered with a physiological saline and a group that was free from a chelating agent (i.e., administered with R-CHI3-DTPA-Gd only). Except the physiological saline-administered group, the dose as R-CHI3-DTPA-Gd was 0.33 mmol/kg.

As shown in Table 4, the groups administered with the specimens mixed with DTPA or DTPA-Ca showed a significant effect of inhibiting production of calculi. The groups administered with the specimens mixed with EDTA-Ca also showed a week effect of the inhibition, considering not only the amount of calculi but also indexes that reflect efficacy, namely the hyperplasia of vesical transitional epithelium and the frequency of urinary occult blood. (The chelating agent-free group is estimated to include individuals that discharged calculi from their urinary bladders, judging from the hyperplasia of vesical transitional epithelium and the frequency of urinary occult blood.)

**Table 4**

| Calculus prevention effects by chelating agents | | | |
|---|---|---|---|
| Administered groups | Calculi | Hyperplasia of vesical transitional epithelium | Urinary occult blood |
| Physiological saline administered group | 0/10 | 0/10 | 6/140 |
| Chelating agent-free group | 3/10 | 3/10 | 21/140 |
| DTPA-10 group | 0/10 | 0/10 | 8/140 |
| DTPA-Ca-10 group | 0/10 | 0/10 | 3/140 |
| EDTA-Ca-1 group | 4/10 | 2/10 | 17/140 |
| EDTA-Ca-10 group | 1/10 | 1/10 | 6/140 |
| (Urinary occult blood: The total number of urinary occult blood occurrences observed in the total number of inspections made every day for 14 days) | | | |

### Example 2

### Calculus prevention effect by DTPA

Toxicity studies by repeated intravenous administration were conducted on male rats for 2 weeks using, as a specimen, R-CHI3-DTPA-Gd to which DTPA was added. In this test, two lots (hereinafter called lot #1 and lot #2) of R-CHI3-DTPA-Gd were provided, and the following administered groups were set up. Specifically, there were groups which were administered with a specimen of lot #1 of R-CHI3-DTPA-Gd mixed with DTPA in an amount equivalent to 1 mmol or 3 mmol per 500 mmol of the DTPA-Gd portion of the R-CHI3-DTPA-Gd, which are respectively called DTPA-1 group #1 and DTPA-3 group #1. Similarly, there were groups which were administered with a specimen of lot #2 of R-CHI3-DTPA-Gd mixed with DTPA in an amount equivalent to 1 mmol, 1.3 mmol or 10 mmol per 500 mmol of the DTPA-Gd portion of the R-CHI3-DTPA-Gd, which are respectively called DTPA-1 group #2, DTPA-1.3 group #2 and DTPA-10 group #2. As control groups, were set up a group that was administered with a physiological saline and a group that was free from a chelating agent (i.e., administered with R-CHI3-DTPA-Cd of lot (1) only). Except the physiological saline-administered group, the dose as R-CHI3-DTPA-Gd was 0.33 mmol/kg.

**Table 5**

| Calculus prevention effect by DTPA | | | |
|---|---|---|---|
| Administered groups | Calculi | Hyperplasia of vesical transitional epithelium | Urinary occult blood |
| Physiological saline -administered group | 0/10 | 0/10 | 0/140 |
| Chelating agent-free group | 6/10 | 7/10 | 34/140 |
| DTPA-1 group #1 | 5/10 | 7/10 | 24/140 |
| DTPA-3 group #1 | 5/10 | 5/10 | 17/140 |
| DTPA-1 group #2 | 5/10 | 6/10 | 27/140 |
| DTPA-1.3 group #2 | 5/10 | 7/10 | 26/140 |
| DTPA-10 group #2 | 3/10 | 4/10 | 19/140 |
| (Urinary occult blood: The total number of urinary occult blood occurrences observed in the total number of inspections made every day for 14 days) | | | |

### Example 3

### Calculus prevention effect by R-CHT3-DTPA

Toxicity studies by repeated intravenous administration were conducted on male rats for 2 weeks using a specimen that contained R-CHI3-DTPA in R-CHI3-DTPA-Gd. The molar ratio of them was R-CHI3-DTPA-Gd:R-CHI3-DTPA = 500:1. The doses were 0.013, 0.067, 0.33 and 1.67 mmol/kg as R-CHI3-DTPA-Gd (0.04, 0.2, 1 and 5 mmol/kg as DTPA-Gd), and 6 rats were used for each dose. No calculus was observed in every dose, and an obvious prevention effect was observed compared with the results of the chelating agent-free groups of Examples 1 and 2.

### Industrial Availability

In image diagnosis or therapy using compounds containing lanthanoid metal ions or other heavy metals, urinary calculi caused by metal ions liberated from said compounds used for image diagnosis or therapy can be prevented by administration of a composition that comprises, as a major ingredient, a multidentate ligand capable of forming a complex with metal ions.

## Claims

1. A composition for preventing urinary calculi, which comprises a multidentate ligand capable of forming a complex with lanthanoid metal ions or other heavy metal ions, or an alkaline earth metal complex of said ligand.

2. A composition according to claim 1, wherein the multidentate ligand is a bifunctional ligand chemically bonded to a physiologically acceptable monosaccharide, oligosaccharide, polysaccharide, amino acid, oligopeptide, polypeptide, nucleotide, oligonucleotide, polynucleotide, protein, protein fragment, chemical derivative thereof, or synthetic polymer.

3. A composition according to claim 2, wherein the bifunctional ligand has a site for forming a complex with a metal, said site comprising a polyaminopolycarboxylic acid or polyaminopolyphosphonic acid.

4. A composition according to claim 1, wherein the multidentate ligand is a polyaminopolycarboxylic acid or polyaminopolyphosphonic acid.

5. A composition according to claim 3 or 4, wherein the polyaminopolycarboxylic acid is ethylenediaminediacetic acid, nitrilotriacetic acid, ethylenediaminetetraacetic acid, diaminocyclohexanetetraacetic acid, diethylenetriaminepentaacetic acid, triethylenetetraminehexaacetic acid, 1,4,7,10-tetraazacyclododecane-1,4,7,10-tetraacetic acid, 1,4,8,11-tetraazacyclotetradecane-1,4,8,11-tetraacetic acid, or derivative thereof.

6. A composition according to claim 3 or 4, wherein the polyaminopolyphosphonic acid is ethylenediaminetetrakismethylenephosphonic acid or derivative thereof.

7. A composition according to claim 1, wherein the multidentate ligand is a bifunctional ligand chemically bonded to a physiologically acceptable amino oligosaccharide or derivative thereof.

8. A composition according to claim 7, wherein the bifunctional ligand has a site for forming a complex with a metal, said site comprising a polyaminopolycarboxylic acid or polyaminopolyphosphonic acid.
